# FASCICULE DE BREVET EUROPEEN

(11) **EP 1 206 482 B1**
(45) Date de publication et mention de la délivrance du brevet: **24.09.2003**
(21) Numéro de dépôt: 00958701.5
(22) Date de dépôt: 18.08.2000
(51) Int. Cl.: C07K 1/00, C07K 1/107

(54) **PROCEDE DE COUPLAGE, EN SOLUTION, ENTRE UN PEPTIDE ET AU MOINS UN AUTRE COMPOSE ET SES APPLICATIONS**
VERFAHREN ZUR KUPPLUNG ZWISCHEN EINEM PEPTID UND MINDESTENS EINER WEITEREN VERBINDUNG IN LÖSUNG UND VERWENDUNGEN DAVON
METHOD FOR COUPLING, IN SOLUTION, A PEPTIDE WITH AT LEAST ANOTHER COMPOUND AND USES THEREOF

(30) Priorité: 19.08.1999 FR 9910626; 06.12.1999 FR 9915342
(43) Date de publication de la demande: 22.05.2002
(73) Titulaire: INSTITUT PASTEUR DE LILLE, 59019 Lille Cédex (FR); CENTRE NATIONAL DE LA RECHERCHE SCIENTIFIQUE (CNRS), 75794 Paris Cedex 16 (FR)
(72) Inventeur: BONNET, Dominique, F-59000 Lille (FR); MELNYK, Oleg, F-59370 Mons en Baroeul (FR); GRAS-MASSE, Hélène, F-59710 Merignies (FR)
(74) Mandataire: Lepeudry-Gautherat, Thérèse
(86) Numéro de dépôt international: FR0002336
(87) Numéro de publication internationale: WO01014408

(56) Documents cités:
- O MELNYK ET AL.: "Synthesis of lipopeptides using hydrazone chemical ligation" JOURNAL OF PEPTIDE RESEARCH., vol. 52, no. 9, septembre 1998 (1998-09), pages 180-184, XP000774264 MUNKSGAARD INTERNATIONAL PUBLISHERS, COPENHAGEN., DK ISSN: 1397-002X cité dans la demande
- C KLINGUER ET AL.: "Synthesis of hydrazinopeptides using solid-phase N-amination. Application to chemical ligation." TETRAHEDRON LETTERS, vol. 37, no. 40, 1996, pages 7259-7263, XP002140473 OXFORD GB cité dans la demande
- D BONNET ET AL.: "Synthesis of hydrazinopeptides using solid-phase N-electrophilic amination: extension to the Fmoc/tert-butyl strategy and chemistry of the N-N bond in strong acid media." JOURNAL OF PEPTIDE RESEARCH., vol. 54, no. 9, octobre 1999 (1999-10), pages 270-278, XP000849311 MUNKSGAARD INTERNATIONAL PUBLISHERS, COPENHAGEN., DK ISSN: 1397-002X
- CHEMICAL ABSTRACTS, vol. 132, no. 17, 24 avril 2000 (2000-04-24) Columbus, Ohio, US; abstract no. 222853, D BONNET ET AL.: "Chemoselective acylation of hydrazinopeptides; a novel and mild method for the derivatization of peptides with sensitive fatty acids." XP002140475 & TETRAHEDRON LETTERS, vol. 41, no. 1, janvier 2000 (2000-01), pages 45-48, OXFORD GB
- O MELNYK ET AL.: "Synthesis of lipopeptides using hydrazone chemical ligation" PEPT. 1998, PROC. EUR. PEPT. SYMP., 25TH MEETING DATE 30-8 TO 6-9-1998. EDITORS: S. BAJUSZ & F. HUDECZ, 1999, pages 108-109, XP002161584 Budapest, HU

## Description

La présente invention est relative à un procédé de couplage, en solution, entre un peptide et au moins un composé portant une fonction acide carboxylique ou alcool, tel qu'un lipide, un sucre, un alcool ou un marqueur de fluorescence, ainsi qu'à des peptides modifiés qui sont essentiellement constitués d'un peptide lié, par un lien hydrazide, à au moins un composé tel que défini ci-dessus.

La présente invention est également relative à l'utilisation de l'acide N,N'-tri(Boc)hydrazinoacétique ou de l'acide N,N'-di(Boc)hydrazinoacétique pour fonctionnaliser un peptide par un groupement α-hydrazinoacétique.

Le problème de l'entrée dans les cellules vivantes de différentes substances dotées de propriétés pharmacologiques revêt une grande importance sur le plan thérapeutique. Les peptides synthétiques et les oligonucléotides traversent difficilement la membrane cellulaire. Une approche intéressante pour améliorer leur capacité à pénétrer dans la cellule est de les modifier par une partie lipophile. Ainsi, il a été montré qu'un peptide modifié par une simple chaîne aliphatique est capable de pénétrer au sein de la cellule par transfert passif au travers de la membrane et d'interagir avec sa cible intracytoplasmique. Les lipopeptides représentent donc des molécules intéressantes pour vectoriser un motif fonctionnel au sein de la cellule.

La synthèse de lipopeptides peut être réalisée, par exemple, en couplant un acide gras à un peptide en phase solide, comme décrit notamment par K. THIAM *et al*. dans *Biochemical and Biophysical Research Communications*, 1998, 253, 639-647. En fin de synthèse, des étapes de clivage de la liaison peptide/support solide et de déprotection des chaînes latérales du peptide par un acide fort doivent être réalisées. Ce traitement limite de façon importante le choix de la partie lipophile ; il interdit notamment l'utilisation d'acides gras insaturés. En outre, la purification des lipopeptides par chromatographie liquide haute performance en phase inverse est difficile et conduit à des rendements faibles, eu égard aux nombreuses impuretés qui sont présentes en fin de synthèse.

Il a également été proposé de coupler, en solution, une protéine à un groupement palmitoyl-coenzyme A, ce dernier étant introduit au niveau du groupement thiol d'une cystéine. Un tel couplage se traduit par la formation d'un lien thioester, qui présente l'inconvénient d'être peu stable. D'autre part, cette stratégie est limitée à la modification de certaines protéines par le palmitoyl-coenzyme A et ne peut pas être généralisée à la synthèse de lipopeptides.

Les stratégies actuelles pour la synthèse de lipopeptides consistent également à mettre en oeuvre des réactions de ligation chimique. La ligation chimique permet de lier, en solution et dans des conditions extrêmement douces, deux structures peptidiques préalablement purifiées et totalement déprotégées.

Il a ainsi été proposé de lier, dans un tampon aqueux, un acide gras à un peptide par un lien disulfure. Cependant, le lien disulfure pose de nombreux problèmes ; un tel lien est en effet peu stable et susceptible d'être dégradé en présence de thiols, d'où la nécessité d'éviter la contamination des solvants utilisés pour la solubilisation des produits par des thiols, ainsi que l'impossibilité d'introduire une cystéine dans la séquence peptidique à vectoriser. L'utilisation de la chimie des thiols nécessite, en outre, de travailler sous atmosphère inerte dans le but d'éviter l'oxydation des thiols.

W. ZENG *et al.* (*J. Pept. Sc.,* 1996, 2, 66-72) ont également proposé de lier, en solution, un peptide totalement déprotégé et préalablement purifié à une structure lipidique polyfonctionnelle liée à un peptide, et ce par le biais d'un lien oxime. La partie lipophile est introduite sur une séquence peptidique en phase solide, une telle méthode présentant les inconvénients mentionnés ci-dessus, à savoir la limitation au niveau du choix de la partie lipophile et les difficultés liées à la purification de la structure lipidique.

De façon similaire, O. MELNYK *et al*. (*J. Peptide Res*., 1998, 52, 180-184) ont décrit la ligation, en solution et par un lien hydrazone, entre un peptide portant une chaîne lipophile et une fonction aldéhyde et un autre peptide modifié au niveau de la chaîne latérale de lysine par un groupement hydrazino. Le lien hydrazone est réalisé en solution, mais le composé lipophile, de nature peptidique, est synthétisé en phase solide et les limitations sont identiques à celles évoquées précédemment.

Par ailleurs, C. KLINGUER *et al*. (Tetrahedron Letters, 1996, 37, n° 40, 7259-7262) ont décrit la ligation, dans un mélange eau/acétonitrile et par un lien hydrazone, entre un peptide portant une fonction hydrazine et le cyclohexane-carboxaldéhyde. Le procédé décrit par ces auteurs ne permet toutefois pas d'obtenir des composés stables et donc utilisables pour la vectorisation de principes actifs : en effet, le lien hydrazone formé entre l'hydrazine et l'aldéhyde est instable dans une large gamme de pH.

La ligation chimique apparaît comme une méthode de choix pour la synthèse de lipopeptides permettant l'amélioration des rendements d'obtention de ces composés. Cependant, nous avons vu qu'il n'existe pas, à l'heure actuelle, de méthodes de ligation n'utilisant pas la chimie des thiols et permettant un couplage direct d'un composé lipophile, non lié à une structure porteuse, à un peptide totalement déprotégé.

Les Inventeurs se sont donc donnés pour but de pourvoir à une nouvelle stratégie de synthèse de lipopeptides et, de façon générale, de peptides modifiés par différents composés de nature lipidique ou autre, par ligation chimique en solution.

Cette nouvelle stratégie de synthèse doit notamment répondre aux critères suivants:
- le couplage du composé sus-mentionné, par exemple un lipide, au peptide s'effectue en solution,
- le couplage s'effectue à partir d'un peptide totalement déprotégé, la réaction étant chimiosélective,
- les conditions réactionnelles du couplage permettent l'utilisation directe d'acides gras et de dérivés du cholestérol commerciaux,
- les conditions réactionnelles du couplage permettent notamment l'introduction, sur le peptide, d'acides carboxyliques et d'alcools sensibles, comme par exemple des acides gras complexes mono- et polyinsaturés et des dérivés du cholestérol,
- le lien formé au cours du couplage est très stable dans une large gamme de pH.

Les Inventeurs se sont également donnés pour but de pourvoir à des peptides modifiés, susceptibles d'être obtenus par ligation chimique, dans lesquels lesdits peptides sont liés à différents composés, notamment à des lipides, par un lien très stable et qui ne présentent pas les inconvénients des liens disulfures de l'Art antérieur.

Ces buts sont atteints par la création d'un lien hydrazide entre le peptide et le composé qui lui est couplé, lors d'une synthèse convergente en solution.

La présente invention a pour objet un procédé de couplage entre un peptide et au moins un composé A, de nature non peptidique, portant une fonction sélectionnée dans le groupe constitué par les fonctions acide carboxylique et les fonctions alcool, caractérisé en ce que ledit couplage comprend une étape de réalisation, en solution, d'un lien hydrazide entre ledit peptide et ledit composé A.

Au sens de la présente invention, on entend par " peptide " tout enchaînement de plusieurs acides aminés, quels que soient leur nature et leur nombre ; le terme "peptide" désigne donc aussi bien des oligopeptides (dipeptides ou tripeptides) que des polypeptides ou des protéines.

De façon particulièrement avantageuse, le procédé selon l'invention, réalisé en solution, permet d'éviter une étape de clivage du peptide modifié obtenu du support, clivage dont nous avons vu précédemment qu'il limite de façon importante le choix du composé couplé audit peptide. En outre, le lien hydrazide réalisé entre le peptide et le (ou les) composé(s) A est très stable, et ce dans une gamme de pH très large, et *in vivo*.

Selon un mode de mise en oeuvre avantageux du procédé de couplage selon la présente invention, ce dernier comprend, pour réaliser ledit lien hydrazide, les étapes suivantes :
a) activation de la fonction portée par ledit composé A en une fonction réactive correspondante, respectivement sélectionnée dans le groupe constitué par les fonctions ester et les fonctions carbonate, lorsque le composé A porte respectivement une fonction acide carboxylique et une fonction alcool ; et
b) réaction, en solution et à un pH inférieur à 6, entre ledit composé A activé obtenu en a) et un peptide, totalement déprotégé, portant au moins un groupement hydrazine ou dérivé d'hydrazine soit au niveau de son extrémité N-terminale, soit au niveau de l'extrémité de la chaîne latérale d'une lysine ou d'une ornithine éventuellement présente à un endroit quelconque de la séquence peptidique.

Au sens de la présente invention, on entend par " groupement hydrazine " un groupement de formule -NH-NH₂. On entend par " groupement dérivé d'hydrazine " tout groupement comprenant au moins le motif -NR-NH₂ (y compris, de façon avantageuse, le motif -CO-CHR₁-NR-NH₂), où R₁ et R représentent, indépendamment l'un de l'autre, un atome d'hydrogène ou un groupement alkyle saturé ou insaturé, linéaire, ramifié ou cyclique, comprenant de 1 à 10 atomes de carbone, ainsi qu'éventuellement 1 à 3 hétéroatomes choisis parmi l'oxygène, le soufre et l'azote, et pouvant être substitué par 1 à 6 groupements choisis parmi les groupes hydroxy, alkoxy, aryloxy, amino, aminoalkyle, aminoaryle, thio, thioalkyle, carbonyle, carboxyle, guanidino et carboxamido.

Le lien hydrazide formé au cours du procédé de couplage conforme à l'invention comprend donc au moins le motif -CO-NH-NR-, R étant tel que défini ci-dessus.

Un groupement hydrazine peut être introduit, soit au niveau de l'extrémité N-terminale du peptide, soit au niveau de l'extrémité de la chaîne latérale d'une lysine ou d'une ornithine éventuellement présente à un endroit quelconque de la séquence peptidique, par tout moyen connu de l'Homme de l'Art, par exemple selon un protocole de N-amination tel que décrit par C. KLINGUER *et al*. dans Tetrahedron Letters, 1996, 37, 40, 7259-7262.

De façon particulièrement avantageuse, la réaction de couplage entre ledit composé A activé et ledit peptide totalement déprotégé, fonctionnalisé tel que décrit ci-dessus, permet d'éviter toute étape de déprotection des chaînes latérales du peptide avec un acide fort postérieurement à la réaction de couplage, ce qui permet d'utiliser, en tant que composé A, des acides gras sensibles. Le procédé selon l'invention permet ainsi d'obtenir directement le peptide modifié, c'est-à-dire le peptide couplé au composé A.

Le procédé selon l'invention permet de réaliser une réaction chimiosélective entre le groupement fonctionnel (groupement hydrazine ou dérivé d'hydrazine) introduit sur le peptide et le (ou les) composé(s) A activés ; en effet, la réaction s'effectue à un pH inférieur à 6, pH tel que les fonctions amines des chaînes latérales des lysines (fonction ε-NH₂) ou des ornithines (fonction δ-NH₂) ou la fonction α-NH₂ N-terminale éventuellement présentes au sein de la séquence peptidique sont protonnées et donc peu réactives. Le contrôle du pH permet donc d'acyler préférentiellement le groupement hydrazine ou dérivé d'hydrazine introduit sur le peptide, sans que les autres groupements fonctionnels des chaînes latérales des acides aminés constitutifs du peptide réagissent.

La réaction de couplage réalisée au cours du procédé selon la présente invention (étape b) s'effectue dans des conditions opératoires très douces et, de façon particulièrement avantageuse, ne nécessite pas de travailler dans des conditions inertes, comme c'est le cas pour certains des procédés de l'Art antérieur, notamment ceux qui consistent à coupler un peptide à un acide gras par un lien disulfure.

Selon un mode de mise en oeuvre avantageux du procédé de couplage selon l'invention, ledit procédé comprend, en outre, une étape c) de purification du peptide modifié obtenu à l'étape b).

Une telle purification est, de façon classique, réalisée par chromatographie liquide haute performance. En comparaison à la purification d'un peptide modifié obtenu par un procédé de couplage réalisé en phase solide, tel que décrit précédemment, la purification du peptide modifié obtenu par le procédé de couplage conforme à la présente invention conduit à des rendements bien meilleurs, le peptide modifié obtenu à l'étape b) étant plus pur qu'un peptide modifié obtenu en phase solide.

Selon un autre mode de mise en oeuvre avantageux du procédé de couplage selon l'invention, après l'étape a) d'activation de la fonction portée par le composé A, la fonction réactive correspondante portée par le composé A est sélectionnée dans le groupe constitué par les esters et les carbonates de succinimidyle, de sulfosuccinimidyle et d'aryle.

A titre d'exemple d'esters et de carbonates d'aryle, on peut citer les esters et les carbonates de para-nitrophényle.

Selon un autre mode de mise en oeuvre avantageux du procédé de couplage selon l'invention, ledit groupement dérivé d'hydrazine portée par le peptide est un groupement α-hydrazinoacétique (groupement de formule -CO-CH₂-NH-NH₂).

Selon une disposition préférée de ce mode de mise en oeuvre, préalablement à l'étape b) du procédé selon l'invention, ledit peptide est fonctionnalisé par un groupement α-hydrazinoacétique, soit au niveau de son extrémité N-terminale, soit au niveau de l'extrémité de la chaîne latérale d'une lysine ou d'une omithine éventuellement présente à un endroit quelconque de la séquence peptidique, à l'aide de l'acide N,N'-tri(Boc)hydrazinoacétique ou de l'acide N,N'-di(Boc)hydrazinoacétique.

Selon une modalité préférée de cette disposition, la fonctionnalisation dudit peptide par un groupement α-hydrazinoacétique, grâce à l'acide N,N'-tri(Boc)hydrazinoacétique ou à l'acide N,N'-di(Boc)hydrazinoacétique, est suivie d'une étape de purification dudit peptide fonctionnalisé par chromatographie liquide haute performance, à l'aide d'un éluant constitué d'un mélange eau/alcool, de préférence un mélange eau/isopropanol, comprenant de l'acide trifluoroacétique Un tel éluant permet avantageusement d'éviter toute dégradation du groupement α-hydrazinoacétique porté par le peptide.

Selon un autre mode de mise en oeuvre avantageux du procédé de couplage selon l'invention, ledit composé A est sélectionné dans le groupe constitué par les lipides, les sucres, les alcools et les marqueurs de fluorescence.

A titre d'exemple de marqueur de fluorescence utilisable, on peut citer, de façon non limitative, la fluorescéine ou la rhodamine.

Selon une disposition préférée de ce mode de mise en oeuvre, lesdits lipides sont sélectionnés dans le groupe constitué par les acides gras saturés, les acides gras insaturés et les stérols. En effet, le procédé selon l'invention permet avantageusement de coupler à un peptide des acides gras complexes (mono- et polyinsaturés) et, de manière générale, tout acide carboxylique sensible. De préférence, les lipides sus-mentionnés sont sélectionnés dans le groupe constitué par l'acide palmitique, l'acide stéarique, l'acide cis-9,10-époxystéarique, l'acide oléique, l'acide linoléique et le cholestérol.

La présente invention a également pour objet un peptide modifié essentiellement constitué d'un peptide lié, par un lien hydrazide, à au moins un composé A sélectionné dans le groupe constitué par les lipides, les sucres, les alcools et les marqueurs de fluorescence.

Selon une disposition préférée de ce mode de réalisation, le peptide modifié selon la présente invention est un oligopeptide essentiellement constitué d'un peptide lié, par un lien hydrazide, à au moins un lipide sélectionné dans le groupe constitué par les acides gras saturés, les acides gras insaturés et les stérols.

De préférence, ledit, oligopeptide selon l'invention est essentiellement constitué d'un peptide lié, par un lien hydrazide, à au moins un lipide sélectionné dans le groupe constitué par l'acide palmitique, l'acide stéarique, l'acide cis-9,10-époxystéarique, l'acide oléique, l'acide linoléique et le cholestérol.

La stabilité du lien hydrazide rend les peptides modifiés solon l'invention particulièrement intéressants, puisque le lien hydrazide est stable aussi bien *in vivo* que dans une gamme de pH très large, En outre, le lien hydrazide est stable dans des conditions d'hydrogénation catalytique, ce qui permet, par exemple dans le cas de peptides modifiés par des acides gras insaturés, la synthèse de lipopeptides marqués au tritium au niveau de la chaîne grasse, utiles pour un suivi radioactif intracellulaire desdits lipopeptides et une meilleurs compréhension de leur mécanisme d'action.

La présente invention a également pour objet un vaccin synthétique et un réactif de diagnostic qui comprennent au moins un peptide modifié selon la présente invention, tel que décrit ci-dessus.

La présente invention a également pour objet l'utilisation du procédé do couplage conforme à l'invention, tel que décrit ci-dessus, pour la préparation, d'un médicament comprenant un principe actif de nature peptidique vectorisé, utile pour le ciblage cellulaire.

La présente invention a, en outre, pour objet l'utilisation de l'acide N,N'-tri(Boc)hydrazinoacétique ou de l'acide N,N'-di(Boc)hydrazinoacétique pour fonctionnaliser un peptide destiné à être couplé conformément au procédé de couplage ci-dessus, dans le cas où la groupement hydrazine porté par le peptide est un groupement α-hydrazinoacétique, et ce préalablement à l'étape b), par un groupement α-hydrazinoacétique, soit au niveau de l'extrémité N-terminals dudit peptide, soit au niveau de l'extrémité de la chaîne latérale d'une lysine ou d'une ornithine éventuellement présente à un endroit quelconque de la séquence peptidique.

Il est bien entendu, toutefois, qu'un groupement α-hydrazinoacétique peut être introduit sur ledit peptide, soit au niveau de l'extrémité N-terminale dudit peptide, soit au niveau de l'extrémité de la chaîne latérale d'une lysine ou d'une ornithine éventuellement présente à un endroit quelconque de la séquence peptidique, par tout procédé connu de l'Homme de l'Art ; par exemple, la fonctionnalisation d'un peptide par un groupement α-hydrazinoacétique peut être réalisée par une réaction de N-amination en phase solide, telle que décrite par C. KLINGUER *et al*. dans Tetrahedron Letters, 1996, 37, 40, 7259-7262, au moyen du réactif commercial N-Boc-3-(4-cyanophényl)oxaziridine (BCPO). C'est le cas, par exemple, d'une réaction de N-amination effectuée au niveau d'un résidu glycine situé en position N-terminale d'un peptide ou au niveau de la chaîne latérale d'une lysine ou d'une ornithine présente à un endroit quelconque de la séquence peptidique.

Toutefois, étant donné le coût élevé du BCPO et le temps très important requis pour une telle réaction, cette voie de synthèse n'est adaptée qu'à la fonctionnalisation de produits à haute valeur ajoutée, synthétisés en faible quantité. De façon particulièrement avantageuse, l'utilisation de l'acide N,N'-tri(Boc)-hydrazinoacétique ou de l'acide N,N'-di(Boc)hydrazinoacétique selon la présente invention est plus simple et bien moins coûteuse pour fonctionnaliser un peptide par un groupement α-hydrazinoacétique. Cette fonctionnalisation est effectuée en phase solide, le peptide fonctionnalisé étant ensuite séparé du support solide et déprotégé selon des méthodes connues de l'Homme de l'Art ; une étape de purification par chromatographie liquide haute performance peut ensuite être effectuée, en utilisant l'éluant eau/alcool tel que décrit précédemment, qui permet avantageusement d'éviter toute dégradation du groupement α-hydrazinoacétique porté par le peptide.

Outre les dispositions qui précèdent, l'invention comprend encore d'autres dispositions qui ressortiront de la description qui va suivre, qui se réfère à des exemples de mise en oeuvre du procédé objet de la présente invention et de synthèses de peptides modifiés selon la présente invention, ainsi qu'aux dessins annexés, dans lesquels :
- la figure 1 illustre la synthèse des acides N,N'-tri(Boc)hydrazinoacétique 4 et N,N'-di(Boc)hydrazinoacétique 4';
- la figure 2 illustre la synthèse d'un hydrazinopeptide 6 à partir d'un peptide 5 et de l'acide N,N'-tri(Boc)hydrazinoacétique ;
- la figure 3 illustre la synthèse des lipopeptides 11, 12, 13, 14, 16 et 18 selon le procédé conforme à la présente invention, à partir de l'hydrazinopeptide 6 et des lipides 7, 8, 9, 10, 15 et 17, Su représentant un groupe succinimidyle;
- la figure 4 illustre la synthèse du lipopeptide 13 par hydrogénation catalytique du lipopeptide 12 ;
- la figure 5 illustre la synthèse du lipopeptide 21 selon le procédé conforme à la présente invention ;
- la figure 6 illustre la synthèse du lipopeptide 23 selon le procédé conforme à la présente invention.

Il doit être bien entendu, toutefois, que ces exemples sont donnés uniquement à titre d'illustration de l'objet de l'invention, dont ils ne constituent en aucune manière une limitation.

Dans les exemples qui suivent, les abréviations suivantes sont utilisées : eq. : équivalents ; Boc: *tert*-butyloxycarbonyl; Boc₂O : éther de di(*tert*-butyloxycarbonyl) ; CH₂Cl₂ : dichlorométhane; AcOH: acide acétique ; AcOEt: acétate d'éthyle ; Na₂SO₄ : sulfate de sodium; KH₂PO₄ : dihydrogénophosphate de potassium ; Na₂HPO₄ : phosphate de disodium ; DMF: diméthylformamide ; DMAP : 4-diméthyl-aminopyridine ; PEG: polyéthylèneglycol; PS: polystyrène ; CDCl₃ : chloroforme deutéré; CD₃CO₂H : acide acétique d₃ ; TFA: acide trifluoroacétique ; Et₂O : diéthyléther; EDT : éthanedithiol; NMP: N-méthylpyrrolidone; THF: tétrahydrofurane; HBTU: N-oxyde d'hexafluorophosphate de N-[(1H-benzotriazol-1-yl) (diméthylamino) méthylène]-N-méthylméthanaminium; HOBt: N-hydroxybenzotriazole ; ^{t}Bu : *tert*-butyle ; DIEA: diisopropyléthylamine; Pmc: 2,2,5,7,8-pentaméthylchroman-6-sulphonyle; Trt: trityle; Fmoc: 9-fluorènylméthoxycarbonyle ; Pbf: 2,2,4,6,7- pentaméthyldihydro-benzofurane-5-sulfonyle ; BOP: benzotriazole-1-yl-oxy-tris(diméthylamino)-phosphoniumhexafluorophosphate ; HPLC: chromatographie liquide haute performance ; RP-HPLC : chromatographie liquide haute performance en phase inverse ; ES-MS : spectrométrie de masse par électronébulisation; TOF: time-of-flight; MALDI: matrix-assisted laser desorption ionisation; RMN: résonance magnétique nucléaire; TOCSY : total correlation spectroscopy; PDMS: Spectrométrie de Masse à Désorption de Plasma; PAL : peptide-amide linker.

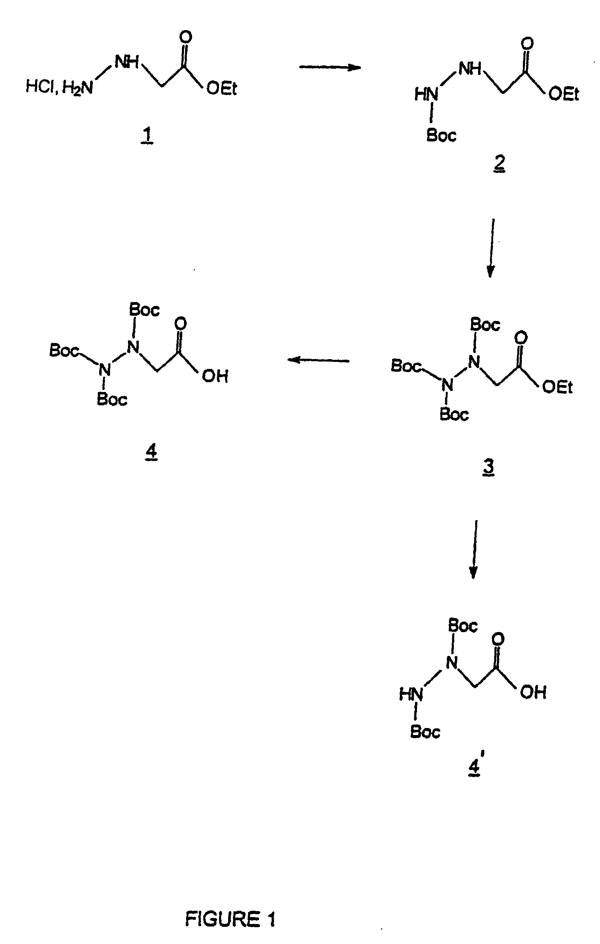

### EXEMPLE 1 : Synthèse des acides N,N'-tri(Boc)hydrazinoacétique 4 et N,N'-di(Boc)hydrazinoacétique 4' (figure 1).

### 1) Synthèse de l'acide N,N'-tri(Boc)hydrazinoacétique 4

### • Synthèse du N-Boc hydrazinoacétate d'éthyle 2

14.9 g (96.4 mmoles) de chlorhydrate d'hydrazinoacétate d'éthyle commercial 1 et 26.1 g (119.5 mmoles) de Boc₂O sont dissous dans 70 ml d'un mélange eau/éthanol (1/1). Après dissolution des réactifs, le milieu réactionnel est refroidi à 0°C. 13.2 ml de N-méthylmorpholine (119.5 mmoles) sont alors ajoutés au milieu réactionnel. Après 15 minutes d'agitation à 0°C, puis 2h à température ambiante, le mélange est dilué dans 100 ml d'eau. La phase aqueuse est saturée avec du KH₂PO₄, puis extraite avec du diéthyléther (3 x 70 ml) et de l'éther de pétrole (2 x 70 ml). Les phases organiques sont réunies puis séchées sur Na₂SO₄ anhydre et enfin concentrées sous pression réduite. L'huile jaune obtenue (19.8 g, 91.1 mmoles, rendement : 94.5%) est séchée sur du pentaoxyde de phosphore (P₂O₅) pendant une nuit. Le produit 2 ainsi obtenu, représenté ci-dessous, est utilisé sans autre forme de purification dans la suite de la synthèse.

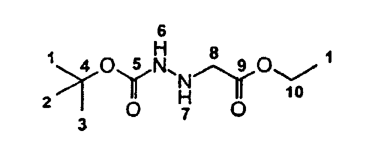

L'analyse du produit 2 est la suivante : RMN ¹H (CDCl₃, réf TMS, 300 MHz, 323 K) δ : 4,19 (q, 2H, H₁₀, J₁₀₋₁₁=7,18 Hz), 4.11 (s, 2H, H₈), 1.45 (m, 9H, H_{1,2,3}), 1.26 (t, 3H, H₁₁, J₁₁₋₁₀=7.16 Hz). RMN ¹³C (CDCl₃, TMS, 75 MHz, 323 K) δ: 175.51 et 174.38 (C₅), 161.76 et 160.83 (C₉), 85.89 et 85.23 (C₄), 65.57 et 65.46 (C₁₀), 57.41 (C₈), 32.87, 32.78 et 32.63 (C₁, C₂, C₃), 18.70 (C₁₁). Analyse élémentaire calculée pour C₉H₁₈N₂O₄ : C 49,53; H 8,31; N 12,84; O 29,32 ; trouvée : C 49,78; H 8,36; N 12,33; O 29,27.

### • Synthèse du N,N'-tri(Boc)hydrazinoacétate d'éthyle 3

Le composé 2 (19.86 g, 91.1 mmoles) est dissous dans 16 ml de CH₂Cl₂ sous atmosphère inerte et en présence de 38.5 ml de Et₃N (276 mmoles) à 0°C. Par ailleurs, 60.2 g (276 mmoles) de Boc₂O sont dissous dans 20 ml de CH₂Cl₂ en présence de 3.4 g (27.6 mmoles) de DMAP à 0°C. Après dissolution totale des réactifs, le mélange composé 2 / Et₃N est additionné gouttes à gouttes au mélange Boc₂O / DMAP sous atmosphère inerte et à 0°C. Une fois l'addition terminée, la température du milieu réactionnel est progressivement ramenée à température ambiante. Après 2h d'agitation, le milieu est dilué avec 50 ml de CH₂Cl₂. La phase organique est lavée avec une solution saturée en KH₂PO₄ (3 x 75 ml), séchée sur sulfate de sodium puis concentrée sous pression réduite. L'huile jaune-orangé résiduelle est purifiée par filtration sur silice (40-60 microns, 160 g) avec un mélange CH₂Cl₂ / AcOEt (97 : 3). L'huile jaune résiduelle est séchée une nuit en présence de P₂O₅. 36.9 g (88.3 mmoles ; rendement : 96.9%) de produit 3 sont ainsi obtenus.

L'analyse du produit 3 par RMN est la suivante : RMN ¹H (CDCl₃, réf TMS, 300 K) δ : 4.16 (s, 2H), 3,71 (q, 2H, J = 7 Hz), 1.46 (m, 27H), 1.23 (t, 3H, J=7 Hz). RMN ¹³C (CDCl₃, réf TMS, 300 K) δ : 167.74 (C=O), 150.48 et 150.23 (C=O), 83.68, 82.46 et 82.00 (C quaternaire), 60.98 et 58.40 (OCH₂CH₃), 53.51 et 51.57 (CH₂CO), 28.09 ((CH₃)₃C), 18.42 et 14.20 (CH₂CH₃).

Analyse élémentaire du produit 3 (formule brute : C₁₉H₃₄N₂O₈) : C 54.53, H 8.19, N 6.69 (calculée), C 54.81, H 8.25, N 6.71 (trouvée).

Analyse du produit 3 par spectrométrie de masse : MALDI-TOF [M+H]⁺ calculé : 419.5, trouvé : 441.4 [M+Na]⁺, 457.4 [M+K]⁺.

### • Synthèse de l'acide N,N'-tri(Boc)hydrazinoacétique 4

Le composé 3 (15.05 g, 36.01 mmoles) est dissous dans 40 ml d'éthanol absolu. Le milieu est refroidi à 0°C et 39.6 ml (39.6 mmoles) d'une solution de soude molaire à 0°C sont ajoutés goutte à goutte. Après 25 minutes d'agitation à 0°C, le milieu réactionnel est neutralisé par ajout, goutte à goutte, de 20 ml d'une solution d'acide citrique (634 mg/ml) jusqu'à un pH de 4.0. Le mélange est ensuite dilué avec 50 ml d'eau et extrait avec du diéthyl éther (2 x 80 ml) puis du dichlorométhane (2 x 80 ml). Les phases organiques sont réunies, lavées avec une solution saturée de NaCI (2 x 40 ml), puis séchées sur Na₂SO₄ anhydre, filtrées et enfin concentrées sous pression réduite. L'huile résiduelle est précipitée dans un mélange diéthyl éther (55 ml)/heptane (74 ml) à froid. Le produit obtenu 4, représenté ci-dessous, est un solide blanc (9.63 g, rendement : 68.5%).

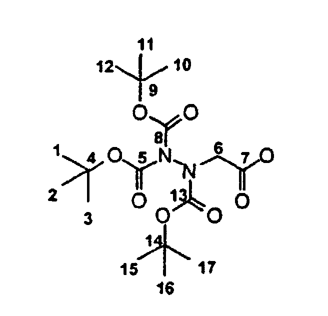

L'analyse du produit 4 est la suivante : RMN ¹H (DMSO-*d*_{*6*}, réf TMS, 300 MHz, 300 K) δ: 4.04 (s, 2H, H₆), 1.42 (m, 27H, H_{1-3,10-12,15-17}), RMN ¹³C (DMSO-*d*_{*6*}, réf TMS, 75 MHz, 300 K) δ: 169.3 (C₇), 153.5 et 149.8 (C_{5,13,8}), 82.9 et 81.7 et 81.1 (C_{4,9,13}), 53.1 et 51.2 (C₆), 27.8 (C_{1-3,10-12,15-17}). Analyse élémentaire calculée pour C₁₇H₃₀N₂O₈ : C 52.30; H 7.74; N 7.17; O 32.78 ; trouvée : C 52.26; H 7.77; N 7.22; O 32.63.

### 2) Synthèse de l'acide N,N'-di(Boc)hydrazinoacétique 4'

Le composé 3 (36.9 g, 88.3 mmoles) dissous dans 135 ml d'éthanol est traité par 135 ml de soude molaire à 0°C. Après 30 minutes d'agitation à 0°C, la température du milieu réactionnel est progressivement ramenée à température ambiante. Le mélange est alors agité 3h30 à température ambiante. Le milieu réactionnel est ensuite dilué avec 110 ml d'eau et extrait avec du diéthyl éther (2 x 80 ml). La phase aqueuse est acidifiée par ajout d'acide chlorhydrique 1 N jusqu'à un pH de 2. La réaction est exothermique. La phase aqueuse est alors extraite avec du dichlorométhane (2 x 80 ml) puis du diéthyl éther (2 x 80 ml). Les phases organiques sont réunies, lavées avec une solution saturée de KH₂PO₄, séchées sur Na₂SO₄ anhydre, filtrées et enfin concentrées sous pression réduite. Le mélange résiduel est conservé pendant une nuit à 4°C puis il est recristallisé dans un mélange diéthyl éther/heptane (90 ml/120 ml) à froid. Le produit obtenu 4', représenté ci-dessous, est un solide blanc (17.9 g, 61.6 mmoles, rendement : 69.8%).

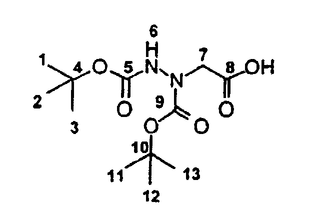

L'analyse du produit 4' est la suivante : RMN ¹H (DMSO-*d*_{*6*}, réf TMS, 300 MHz, 300 K) δ: 9.23 (s, 0.53H, H₆), 9.16 (s, 0.26H, H₆), 8.84 (s, 0.14H, H₆), 8.74 (s, 0.07H, H₆), 3.97 (s, 2H, H₇), 1.39 (m, 18H, H_{1-3,11-13}). RMN ¹³C (DMSO-*d*_{*6*}, réf TMS, 75 MHz, 300 K)δ: 170.10 (C₈), 155.04 et 154.19 (C_{5,9}), 80.15 et 79.54 (C_{4,10}), 54.6 et 53.1 (C₇), 27,93 (C₁₋₃, C₁₁₋₁₃). Analyse élémentaire calculée pour C₁₂H₂₂N₂O₆ : C 49,65; H 7.64; N 9.65; O 33.07 ; trouvée : C 50.09; H 7.84; N 9.57; O 32.64.

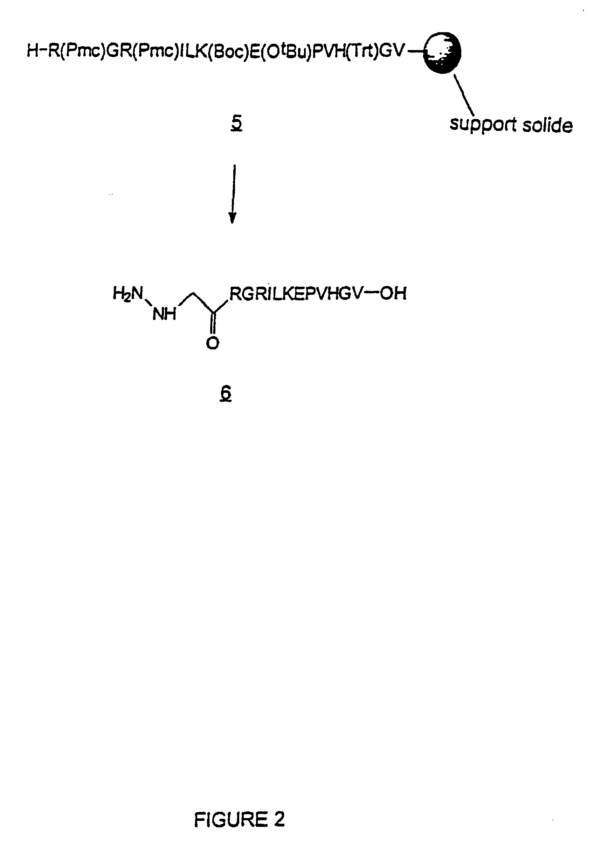

### EXEMPLE 2 : Synthèse et purification de l'hydrazinopeptide 6 (figure 2).

### • Synthèse de l'hydrazinopeptide 6

Le peptide 5 est élaboré sur une résine Wang (0.73 mmol/g, Applied Biosystems, Foster City, USA), selon la stratégie Fmoc/*tert*-butyle, telle que décrite par exemple par FIELDS *et al*. dans *Int. J. Pept. Protein*, 1990, 35, 161, et une activation HBTU/HOBt (voir SCHNÖLZER *et al* dans *Int. J. Pept. Protein Res*., 1992, 40, 180), à l'aide d'un synthétiseur de peptides Applied Biosystem 431A (Foster City, USA). Les protections des chaînes latérales sont : His(Trt), Glu(O^{t}Bu), Arg(Pmc), Lys(Boc). En fin de synthèse, le groupement Fmoc de la fonction α-NH₂ de l'arginine est déplacé en présence de pipéridine 20% dans le DMF. L'acide N,N'-tri(Boc)hydrazinoacétique 4 (1.2 eq) est ensuite introduit manuellement en utilisant l'activation BOP *in situ* (BOP 1.2 eq, DIEA 3.6 eq dans le DMF durant 20 min), comme décrit par exemple par GAIRI *et al*. dans Tetrahedron Letters, 1990, 50, 7363. On pourrait également, en variante, utiliser l'acide N,N'-di(Boc)-hydrazinoacétique. La peptidyl-résine est lavée successivement avec du DMF, du dichlorométhane, puis de l'éther. Elle est ensuite séchée sous pression réduite durant 30 min.

La coupure du lien peptide-résine ainsi que la déprotection des chaînes latérales sont réalisées en présence d'un mélange TFA/H₂O/anisole (1 g de résine sèche/9.5 ml de TFA/0.25 ml d'anisole/0.25 ml d'H₂O) sous agitation durant 2h à température ambiante. Le peptide 6 est précipité dans un mélange Et₂O/heptane (1/1) préalablement refroidi à 0° C (200 ml). Le précipité est centrifugé puis dissous dans un mélange H₂O/AcOH (5/1), congelé et lyophilisé.

### • Purification de l'hydrazinopeptide 6

L'hydrazinopeptide 6 a été purifié par HPLC sur une colonne C18 hyperprep en utilisant un gradient linéaire de 0% à 50% d'un mélange TFA/eau/isopropanol (rapport eau/isopropanol de 2/3, le mélange comprenant 0,05% de TFA) dans un mélange 0.05% TFA/eau. Un tel éluant permet avantageusement d'éviter toute dégradation du peptide. Le composé purifié est lyophilisé et stocké à -20°C.

La pureté du composé purifié est contrôlée par HPLC analytique sur une colonne C18 Vydac en utilisant le même système éluant que précédemment. L'identité du peptide 6 a été vérifiée par analyse ES-MS sur un spectromètre Micromass Quatro ([M+H]⁺ calculé 1432.5, trouvé 1432.7).

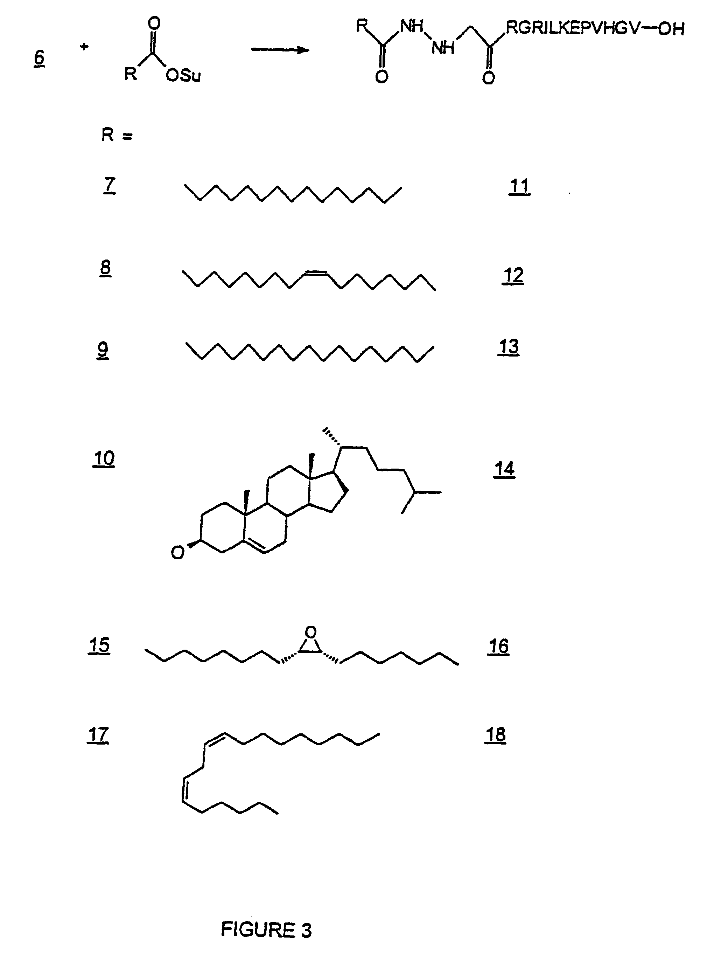

### EXEMPLE 3 : Synthèse des lipopeptides 11, 12, 13, 14, 16 et 18 (figure 3).

### 1) Synthèse des composés 8, 9, 10, 15 et 17

### • Synthèse des composés 8, 9, 15 et 17.

Dans le cas où R (figure 3) représente la chaîne grasse d'un acide oléique, 10 mg (35.4 µmoles) d'acide oléique, 4.08 mg (35.4 µmoles) de N-hydroxysuccinimide et 4.3 µl (27.2 µmoles) de diisopropylcarbodiimide sont dissous dans un mélange THF/dichlorométhane (175 µl / 175 µl). Après une nuit à 0°C, le milieu est concentré sous pression réduite. L'huile résiduelle (composé 8) est reprise dans 6.8 ml de 2-méthyl-propane-2-ol.

On procède de la même manière pour activer les acides stéarique, linoléique et cis-9,10-époxystéarique, c'est-à-dire pour obtenir ces acides sous forme d'esters de succinimidyle (obtention des composés 9, 17 et 15).

### • Synthèse du composé 10.

500 mg (1.13 mmoles) de chloroformiate de cholestéryle et 140.9 mg (1.22 mmoles) de N-hydroxysuccinimide sont dissous dans 2 ml de dichlorométhane à température ambiante. 170 µL (1.22 mmoles) de triéthylamine sont ajoutés au milieu réactionnel. La réaction est exothermique et il se forme un précipité blanchâtre. Après 45 min d'agitation à température ambiante, le milieu est dilué avec 50 ml de dichlorométhane et lavé avec une solution de KH₂PO₄ saturée. La phase organique est séchée sur sulfate de sodium, filtrée puis concentrée sous pression réduite. Le composé 10 obtenu est un solide blanc (451.6 mg, 0.85 mmoles, rendement: 76%). Il s'agit d'un carbonate de cholestéryle activé par le N-hydroxysuccinimide.

### 2) Synthèse du lipopeptide 11

6 mg (3 µmoles) de l'hydrazinopeptide 6, dont la synthèse a été décrite dans l'exemple 2, sont dissous dans 900 µl d'un tampon phosphate/citrate 0.25 mM, pH=5.2 (160.2 µl d'une solution de Na₂HPO₄ 0.2 M et 139.8 µl d'acide citrique 0.1 M complété à 1.2 ml avec de l'eau). Le pH de l'hydrazinopeptide 6 en solution est réajusté si nécessaire avec la solution de Na₂HPO₄ 0.2 M. 1.48 mg (3.6 µ moles) de palmitate de succinimidyle 7 (Sigma) sont dissous dans 900 µl de 2-méthylpropane-2-ol. Les deux solutions sont ensuite mélangées et agitées à température ambiante durant 72 h.

L'utilisation d'un milieu mixte tampon/2-méthyl-propane-2-ol permet à la fois de contrôler le pH du milieu réactionnel et d'assurer la bonne solubilité de l'hydrazinopeptide 6, de l'acide gras 7 et du lipopeptide final 11. De plus, l'introduction de la partie lipophile sur le peptide se fait dans des conditions douces, autorisant ainsi l'introduction d'acides gras sensibles aux acides forts.

L'avancement de la réaction est suivi par HPLC sur une colonne C3 Zorbax (0 à 100% de solvant B à 0.05% TFA/80% acétonitrile/20% eau en 30 mn puis 5 min à 100% de solvant B, 1 ml/mn, détection à 215 nm). Après 72 h, le suivi par HPLC révèle que la réaction est terminée. Le milieu réactionnel est alors dilué avec 5 ml d'un mélange eau/acide acétique (80/20) et purifié sur une colonne C3 Zorbax en utilisant le système éluant précédent. Après congélation et lyophilisation, le lipopeptide 11 est obtenu avec un rendement de 61% (3.89 mg, 1.83 µmoles). On obtient seulement 6% de lipopeptide diacylé (couplage du groupement palmityle non seulement sur le groupement hydrazine du peptide 6, mais aussi sur la fonction amine située sur la chaîne latérale du résidu lysine dudit peptide).

Le lipopeptide 11 purifié est analysé par ES-MS (Micromass Quatro II Electrospray Mass Spectrometer). [M+H]⁺ calculé : 1672.1, trouvé : 1671.6.

### 3) Synthèse des lipopeptides 12, 13, 14, 16 et 18

On procède de façon similaire à ce qui a été décrit en 2) pour la synthèse du lipopeptide 11, en faisant réagir l'hydrazinopeptide 6 avec, respectivement, les composés 8, 9, 10, 15 et 17.

Seule la purification du lipopeptide 16 varie. Sa purification par HPLC est réalisée à pH 7.0 sur une colonne C3 Zorbax en utilisant l'éluant suivant : de 100% de solvant A (tampon phosphate 50 mM, pH 7.0) à 100% de solvant B (tampon phosphate 50 mM, pH 7.0, comprenant 50% d'isopropanol) en 100 minutes, à raison de 3 ml/minute et à 50°C, la détection s'effectuant à 215 nm. Le composé 16 ainsi obtenu est ensuite dessalé en utilisant les conditions suivantes: colonne polystyrène-divinylbenzène, gradient de 100% de solvant A (eau comprenant 0,05% de triéthylamine) à 100% de solvant B (mélange eau/acétonitrile 20/80 comprenant 0,05% de triéthylamine) en 10 minutes, à raison de 4 ml/minute et à 50°C, la détection s'effectuant à 215 nm.

La caractérisation des lipopeptides 12, 13, 14, 16 et 18 par ES-MS et les rendements obtenus pour les différents lipopeptides sont les suivants (tableau I) :

**Tableau I**

| lipopeptide | groupe lipophile | [M+H]⁺ calculé | [M+H]⁺ trouvé | rendement |
|---|---|---|---|---|
| 12 | oléyle | 1697.2 | 1697.8 | 53% |
| 13 | stéaryle | 1699.2 | 1699.5 | 65% |
| 14 | cholestéryle | 1845.6 | 1845.7 | 56% |
| 16 | cis-9,10-époxystéaryle | 1713.2 | 1713.5 | 53% |
| 18 | linoléyle | 1695.2 | 1695.5 | 51% |

On obtient seulement 6, 7 et 8% respectivement de lipopeptides diacylés lors des synthèses des lipopeptides 12, 13 et 14.

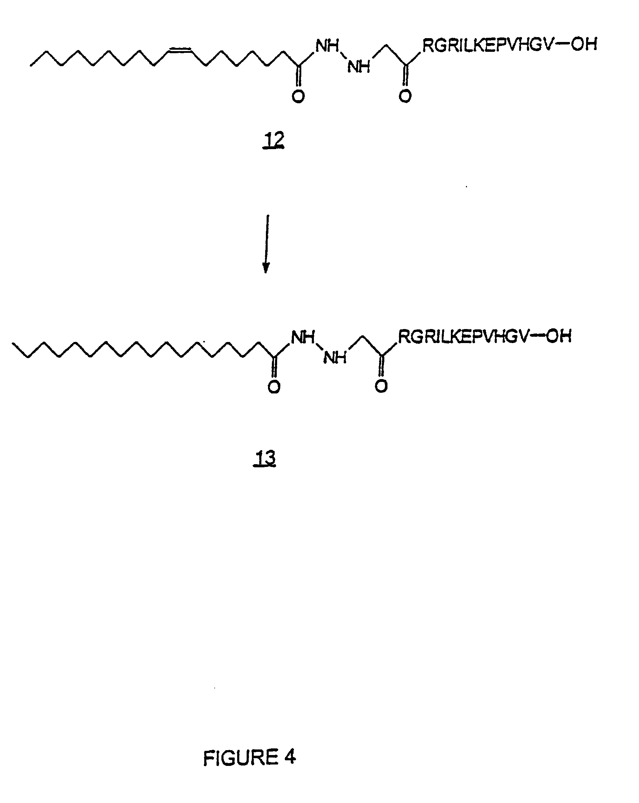

### EXEMPLE 4 : Synthèse du lipopeptide 13 par hydrogénation catalytique du lipopeptide 12 (figure 4).

500 µg de palladium sur charbon à 10% en suspension dans 600 µl d'une solution à 20% d'acide acétique concentré dans l'eau sont ajoutés à 5 mg (2.3 µmoles) du composé 12, obtenu de la façon décrite dans l'exemple précédent, dissous dans 300 µl de la même solution. Après 4 h d'agitation à température ambiante sous atmosphère d'hydrogène, 1.64 mg de palladium sur charbon en suspension dans 100 µl d'acide acétique glacial sont ajoutés au milieu réactionnel. Après 20 h, la conversion est totale et le milieu est filtré sur celite et lavé avec une solution à 20% d'acide acétique dans l'eau (3 x 3 ml), puis du méthanol (3 x 3 ml). Le filtrat est concentré sous pression réduite, congelé puis lyophilisé. Le composé ainsi obtenu est purifié par HPLC sur une colonne C3 Zorbax en utilisant un gradient linéaire de 0% à 55% d'un mélange eau/acétonitrile/TFA (1/4 en eau/acétonitrile, avec 0,05%de TFA) dans un mélange 0.05% TFA/eau (eau comprenant 0,05% de TFA). Le composé purifié (2.55 mg, 1.2 mmoles, rendement : 52%) est lyophilisé et stocké à -20%.

La pureté du composé purifié est contrôlée par HPLC analytique sur une colonne C3 Zorbax en utilisant le même système éluant que précédemment. Le composé est identifié par ES-MS : [M+H]⁺ calculé : 1699.2, trouvé : 1699.6.

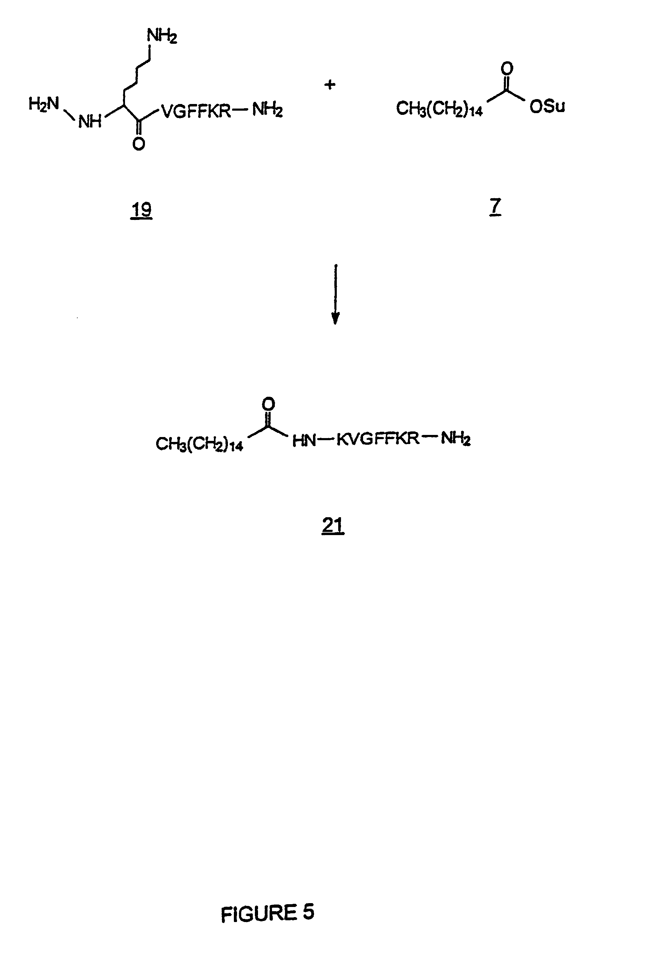

### EXEMPLE 5 : Synthèse du lipopeptide 21 (figure 5).

### 1) Synthèse de l'hydrazinopeptide 19.

L'hydrazinopeptide 19 a été synthétisé sur 0,25 mmol (357.1 mg) de résine Rink Amide aminométhyl-polystyrène comprenant 1% de divinylbenzène (0,70 mmol/g, 100-200 Mesh, Senn Chemicals AG) en utilisant la stratégie Fmoc/*tert*-butyle telle que décrite par exemple par FIELDS *et al. Int. J. Pept. Protein*, 1990, 35, 161, et une activation HBTU/HOBt (SCHNÖLZER *et al, Int. J. Pept. Protein Res*., 1992, 40, 180), à l'aide d'un synthétiseur de peptides Applied Biosystem 431A (Foster City, USA). Les groupements protecteurs Fmoc sont éliminés à l'aide d'une solution de pipéridine à 20% dans le DMF.

La fonction α-NH₂ est modifiée en utilisant la procédure de N-amination électrophile en phase solide mise au point par C. KLINGUER *et al*. (Tetrahedron Letters, 1996, 37, 40, 7259-7262). L'hydrazinopeptide obtenu est déprotégé et coupé de la résine en utilisant 10 ml d'une solution de TFA (94% TFA, 2,5% H₂O, 2,5% thioanisole, 1% triisopropylsilane) pendant 1h30 sous agitation. Le composé est ensuite précipité dans 100ml d'une solution Et₂0/pentane 1/1. Après précipitation et élimination du surnageant, le culot est dissous dans de l'acide acétique à 10%, congelé et lyophilisé.

L'identité de l'hydrazinopeptide 19 est vérifiée par PDMS-TOF sur un Spectromètre de Masse à Désorption de Plasma Bio-ion 20. [M+H]⁺ calculé: 895.5, observé : 895.9.

La purification de l'hydrazinopeptide 19 est réalisée sur une colonne préparative C3 Zorbax (30°C, détection à 235 nm, tampon A= H₂O 100%/TFA 0,05%, tampon B= alcool isopropylique 40%/H₂O 60%/TFA 0,05%, débit de 2 ml/minute, de 0 à 70% en B en 70 minutes). Après congélation et lyophilisation, l'hydrazinopeptide 19 est obtenu avec un rendement de 56%. La pureté du produit après lyophilisation est vérifiée par RP-HPLC dans les mêmes conditions qu'indiquées précédemment.

### 2) Synthèse du lipopeptide 21.

5.06 mg d'hydrazinopeptide 19 sont dissous dans 791 µl de tampon citrate-phosphate pH 5.11. 1.1 eq. (4,12 µmol) de palmitate de succinimidyle 7 (Su représentant un groupe succinimidyle) dissous dans 791 µl de ^{t}BuOH sont alors ajoutés. Le suivi de la réaction est réalisé par RP-HPLC sur une colonne C3 Zorbax. Après 48 h, le milieu réactionnel est purifié sur colonne préparative C3 Zorbax (30°C, détection à 215 nm, tampon A = H₂O 100%/TFA 0,05%, tampon B = acétonitrile 80%/H₂O 20%/TFA 0,05%, débit de 3 ml/minute, de 0 à 70% en B en 70 minutes). Le rendement en lipopeptide 21 est de 60%.

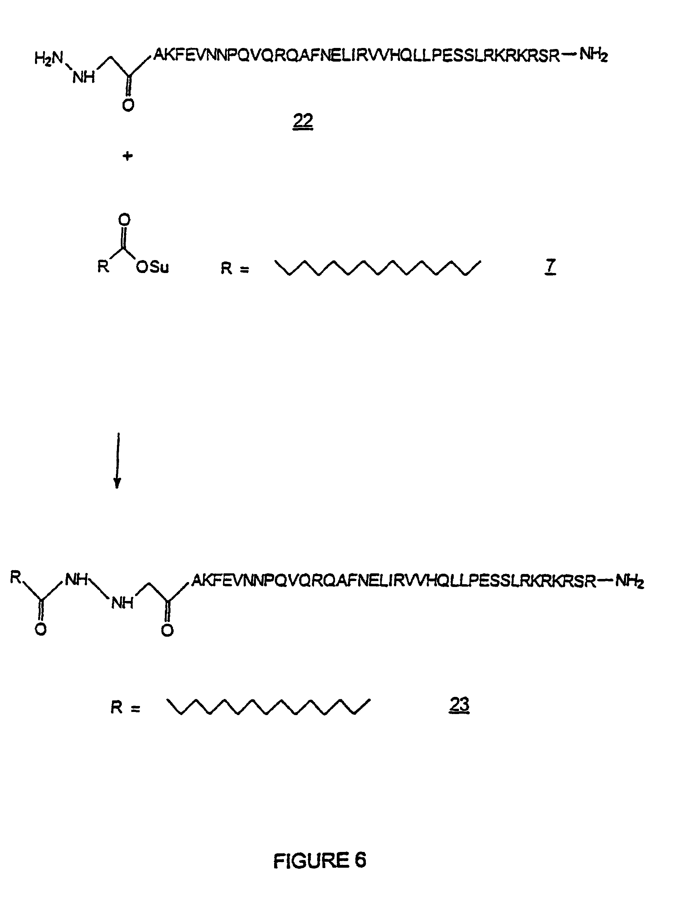

### EXEMPLE 6 : Synthèse du lipopeptide 23 (figure 6).

### 1) Synthèse de l'hydrazinopeptide 22.

Le peptide 22 est élaboré sur une résine Fmoc-PAL-PEG-PS (0,16 mmol/g, Perseptive) selon la stratégie Fmoc/*tert*-butyle et une activation HBTU/HOBt (voir l'exemple 2) sur un synthétiseur de peptides Pioneer-Perseptive. Les protections des chaînes latérales des acides aminés sont les suivantes : His(Trt), Asn (Trt), Glu(O^{t}Bu), Arg(Pbf), Lys(Boc), Ser(^{t}Bu). En fin de synthèse, le groupement Fmoc de la fonction α-NH₂ de l'alanine est éliminé en présence de pipéridine à 20% dans le DMF. L'acide N,N'-tri(Boc)hydrazinoacétique (1,2 eq.) est ensuite introduit manuellement en utilisant l'activation BOP *in situ* (BOP : 1,2 eq., DIEA : 3,6 eq. dans le DMF durant 20 minutes). La peptidyl-résine est lavée successivement avec du DMF, du dichlorométhane, puis de l'éther. Elle est ensuite séchée sous pression réduite pendant 30 minutes. La coupure du lien peptide-résine ainsi que la déprotection des chaînes latérales sont réalisées en présence d'un mélange TFA/phénol/éthanedithiol/thioanisole/H₂O (1 g de résine sèche / 10 ml de TFA / 0,25 ml d'éthanedithiol / 0,25 ml d'H₂O / 0,25 ml de thioanisole / 0,75 g de phénol) sous agitation durant 3h30 à température ambiante. Le peptide est précipité dans 200 ml d'un mélange Et₂O/heptane (1/1) préalablement refroidi à 0°C. Le précipité est centrifugé puis dissous dans un mélange H₂O/AcOH (5/1), congelé et lyophilisé. On obtient 263 mg de peptide brut à partir de 0,072 mmole de résine.

L'hydrazinopeptide 22 a été purifié par HPLC sur une colonne C3 Zorbax en utilisant un gradient linéaire de 0% à 50% en 70 minutes d'un mélange 0,05% TFA/eau/isopropanol (2/3) dans un mélange 0,05% TFA/eau. Le composé purifié (43 mg) est lyophilisé et stocké à -20°C. L'analyse de l'hydrazinopeptide 22 par ES-MS est la suivante : [M+H]⁺ calculé : 4645.5, trouvé : 4645.7. Son analyse en acides aminés est la suivante : Lys(3) : 2,8 ; Arg(6) : 6,0 ; Glu(7) : 7,3 ; Ala(2) : 1,9.

### 2) Synthèse du lipopeptide 23.

Le lipopeptide 23 est obtenu à partir du composé 7 et de l'hydrazinopeptide 22, selon le mode opératoire décrit précédemment pour la synthèse du lipopeptide 11. Il est obtenu avec un rendement de 40%, après purification. L'analyse par ES-MS (Micromass Quatro II Electrospray Mass Spectrometer) du lipopeptide 23 donne les résultats suivants : [M+H]⁺ calculé : 4883.5, trouvé : 4883.7.

### EXEMPLE 7 : Analyse et quantification de l'expression des molécules de classe II du Complexe Majeur d'Histocompatibilité (CMH), à la surface des cellules, induite par incubation de ces cellules avec des peptides modifiés conformes à l'invention.

### 1) Peptides et lipopeptides utilisés.

On utilise les peptides dénommés Mu-Gly, MuSc-Gly, 22 et 22Sc, ainsi que les lipopeptides Mu-Gly-palm, MuSc-Gly-palm, 23 et 23Sc. Le terme «Sc» désigne une version « scramble » du peptide, à savoir une séquence peptidique dans laquelle les acides aminés (leur ordre dans la séquence) ont été mélangés. Le terme « palm» désigne un groupement palmitoyle.

Le peptide 22 et le lipopeptide 23 ont été préparés comme indiqué dans l'exemple 6. Le peptide 22Sc (version « scramble » du peptide 22) présente la séquence suivante :

H₂N-NH-CH₂CO-PSRENQNAVKIQKLSVVLRREQKHRVERLAFRNQSLPF-NH₂

Le peptide 22Sc a été préparé sur une résine Rink amide MBHA PS (0,25 mmole, 0,74 mmol/g, Applied Biosystems, Foster City, USA) selon la stratégie Fmoc/*tert*-butyle, sur un synthétiseur de peptides Applied Biosystem 430 A. A la fin de la synthèse et après déprotection du groupement amino N-terminal, la moitié de la peptidyl-résine (125 µmoles) est traitée en présence de 63,4 mg (162 µmoles) d'acide N,N'-tri(Boc)hydrazinoacétique, de 84,6 mg (162 µmoles) de PyBOP et de 85,2 µl (486 µmoles) de DIEA dans le DMF pendant 30 minutes. La coupure et la déprotection du peptide 22Sc a été réalisée en présence d'un mélange TFA/H₂O/phénol/EDT/thioanisole (1g de résine sèche/10,0 ml de TFA/0,5 ml d'H₂O/0,75g de phénol/0,25 ml d'EDT/0,5 ml de thioanisole). Après précipitation dans un mélange éther/heptane (1/1), centrifugation, congélation et lyophilization, 22Sc a été purifié par RP-HPLC comme le peptide 22. Une fois lyophilisé, le peptide 22Sc (117,5 mg, 15,9%) a été stocké à -20°C. Son analyse en acides aminés est la suivante : Lys(3) : 2,6 ; Arg(6): 6,0 ; Glu(7) : 7,4 ; Ala(2) : 1,9. Son analyse par ES-MS est la suivante : [M+H]⁺ calculé 4645,4 ; trouvé 4645,6.

Le peptide 23Sc (version « scramble » du peptide 23) présente la séquence suivante : palm-NH-NH-CH₂CO-PSRENQNAVKIQKLSVVLRREQKHR-VERLAFRNQSLPF-NH₂. Le peptide 23Sc a été préparé de la même manière que le peptide 23, mais à partir du peptide 22Sc au lieu du peptide 22. Le couplage a été effectué à l'aide de 15,27 mg de peptide 22Sc. Le peptide 23Sc a été purifié sur une colonne C3 Zorbax préparative en utilisant un gradient linéaire de 20 à 60%, en 100 minutes, d'un solvant B (acétonitrile 80%/eau 20%/TFA 0,05%) dans un solvant A (eau/TFA 0,05%). La température est de 50°C, le débit d'élution est de 3 ml/minute et la détection est effectuée à 215 nm. Après congélation et lyophilisation, 2,46 mg (rendement de 16%) de peptide 23Sc ont ainsi été obtenus. La pureté de ce produit purifié est contrôlée par RP-HPLC sur une colonne C3 Zorbax en utilisant un gradient de 0 à 100%, en 60 minutes, du solvant B dans le solvant A (1 ml/minute, 215 nm, 50°C). Un contrôle croisé est réalisé par électrophorèse capillaire en utilisant un tampon citrate 20 mM, pH 3,0 à 40°C, 30 kV sur 10 minutes. L'analyse du produit 23Sc par ES-MS est la suivante : [M+H]⁺ calculé 4883,8 ; trouvé 4882,5.

Les peptides et lipopeptides Mu-Gly, MuSc-Gly, Mu-Gly-palm et MuSc-Gly-palm présentent respectivement les séquences suivantes :

Mu-Gly : H₂NCH₂CO-AKFEVNNPQVQRQAFNELIR-
VVHQLLPESSLRKRKRSR-NH₂

MuSc-Gly : H2N-CH2CO-PSRENQNAVKIQKLSVVLR-
REQKHRVERLAFRNQSLPF-NH2

Mu-Gly-palm : palm-NHCH2CO-AKFEVNNPQVQRQAFNELIR-
VVHQLLPESSLRKRKRSR-NH2

MuSc-Gly-palm : palm-NHCH2CO-PSRENQNAVKIQKLSVVLR-
REQKHRVERLAFRNQSLPF-NH2

Ces peptides ont été obtenus par synthèse peptidique en phase solide selon des protocoles standards, en utilisant la stratégie Fmoc/*tert*-butyle. Pour les lipopeptides Mu-Gly-palm et MuSc-Gly-palm, apres l'élongation de la séquence peptidique et la déprotection de la glycine en position N-terminale avec de la pipéridine à 20% dans le DMF, l'acide palmitique (4 eq.) a été couplé en utilisant une activation HBTU/HOBt/DIEA : 4 eq / 4 eq / 12 eq (équivalents par rapport aux fonctions amines) dans le DMF pendant 40 minutes. La coupure et la déprotection des peptides a été réalisée en présence d'un mélange TFA/H₂O/phénol/EDT/thioanisole (1g de résine sèche/10,0ml de TFA /0,5 ml d'H₂O/0,75g de phénol/0,25 ml d'EDT/0,5 ml de thioanisole). Les peptides et lipopeptides ont été purifiés avant utilisation.

Ainsi, les peptides 22, 23, Mu-Gly et Mu-Gly-palm comprennent la même séquence peptidique de 38 acides aminés. Ils diffèrent en ce qu'ils comprennent ou non une chaîne grasse d'acide palmitique, et au niveau de la nature de la liaison chimique de la chaîne grasse à la séquence peptidique.

### 2) Protocole de quantification de l'expression des molécules de classe II du Complexe Majeur d'Histocompatibilité (CMH) à la surface des cellules.

Les cellules COLO 205 (lignée cellulaire humaine issue d'un carcinome de colon) proviennent de la banque de cellules ATCC (American Type Culture Collection). Elles sont cultivées en milieu RPMI 1640 (Gibco BRL, Courbevoie, France) avec 10% de sérum de veau foetal (SVF) et 5 mM de pyruvate de sodium, et incubées à 37°C en présence de 5% de CO₂. Les cellules sont stimulées pendant 24 heures avec différentes concentrations (35, 50 ou 65 µM) de peptides ou de lipopeptides. Les cellules sont ensuite marquées pendant 1 heure à 4°C avec 10 µl d'anticorps de souris anti-HLA DR de classe-II, couplé à la FITC (clone TAL, 1B5, Cymbus Biotechnology Ltd, Hants, Grande-Bretagne) dans 10% de SVF/PBS, puis lavées 3 fois par du SVF/PBS.

L'expression de surface des molécules de classe II du CMH est analysée par cytométrie de flux sur un cytomètre Coulter EPICS II, à raison de 10 000 évènements par échantillon. L'intensité de fluorescence observée est directement proportionnelle à la quantité de molécules de classe II du CMH présente à la surface des cellules. La moyenne de fluorescence observée pour les cellules non traitées et pour les cellules traitées (colonne « Moyenne» dans le tableau II) permet de calculer un rapport entre la moyenne de fluorescence des cellules traitées et la moyenne de fluorescence des cellules non traitées (colonne « Rapport » dans le tableau II).

### 3) Résultats.

Les résultats obtenus sont rassemblés dans le tableau II.

**Tableau II**

| **Nom du produit** | **35 µM** | | **50 µM** | | **65 µM** | |
|---|---|---|---|---|---|---|
| | **Moyenne** | **Rapport** | **Moyenne** | **Rapport** | **Moyenne** | **Rapport** |
| Mu-Gly-palm | 6,53 | **8,9** | 6,43 | **8,45** | 2,71 | **3,6** |
| MuSc-Gly-palm | 7,23 | **9,86** | 7,45 | **10,16** | 9,76 | **13,31** |
| Mu-Gly | 1,40 | **1,9** | 1,31 | **1,78** | 1,93 | **2,6** |
| MuSc-Gly | 1,47 | **2** | 1,69 | **2,3** | 1,82 | **2,48** |
| 23 | 7,35 | **10,2** | 8,02 | **10,94** | 13,3 | **18,14** |
| 23Sc | 4,72 | **9,16** | 4,51 | **6,15** | 5,55 | **7,5** |
| 22 | 1,13 | **1,5** | 1,11 | **1,5** | 1,25 | **1,70** |
| 22Sc | 1,36 | **1,8** | 1,60 | **2,18** | 1,67 | **2,27** |

On remarque que les produits non dérivatisés par une chaîne palmitique (peptides Mu-Gly et 22) ne conduisent pas à une augmentation de l'expression de CMH II, comparé aux versions « scramble », à savoir MuSc-Gly et 22Sc, respectivement. En revanche, le lipopeptide 23 induit une expression de CMH II dose dépendante, comparé à la version « scrambie » 23Sc. Cette expression est donc spécifique de la séquence et de la présence de la chaîne lipophile liée au peptide via un lien hydrazide. Ainsi, le couplage en solution d'un hydrazinopeptide avec un acide gras activé pour l'obtention du lipopeptide correspondant, conduit à un produit qui est capable de passer la membrane des cellules et d'atteindre une cible intracytoplasmique.

Le lipopeptide 23 donne des résultats supérieurs au lipopeptide synthétisé de manière standard (Mu-Gly-palm). D'autres expériences ont montré que les résultats étaient comparables entre les lipopeptides 23 et Mu-Gly-palm. Cependant, le lipopeptide 23 a une pureté bien supérieure à celle du lipopeptide Mu-Gly-palm. De plus, l'inspection des cellules traitée par les lipopeptides montre que le lipopeptide 23 conforme à l'invention est beaucoup moins cytotoxique pour les cellules que le peptide standard Mu-Gly-palm.

### EXEMPLE 8: Utilisation de l'acide N,N'-di(Boc)hydrazinoacétique pour fonctionnaliser un peptide par un groupement α-hydrazinoacétique

La peptidyl-résine de formule Fmoc-G-R(Pmc)-K(Boc)-R(Pmc)-S(tBu)-H(Trt)-A-G-Y(tBu)-Q(Trt)-T(tBu)-I-O-résine a été synthétisée sur support solide selon la stratégie Fmoc/*tert*-butyle en utilisant une résine Wang, telle que décrite dans l'exemple 2. 143,9 mg (33,7 µmol) de cette peptidyl-résine sont traités, pendant 2 minutes puis pendant 20 minutes, par le mélange pipéridine/NMP (20/80) afin de déprotéger la fonction amine terminale.

11,7 mg d'acide N,N'-di(Boc)hydrazinoacétique (40,4 µmol, 1,2 eq.) sont solubilisés dans 500 µl de NMP en présence de 5,5 mg d'HOBt (40,4 µmol, 1,2 eq.) et de 15,3 mg d'HBTU (40,4 µmol, 1,2 eq.). L'addition de 21 µl de DIEA conduit à l'ester d'hydroxybenzotriazole activé non isolé et immédiatement ajouté à la peptidyl-résine. Après 45 minutes de couplage, le test de Kaiser *(Anal. Biochem*., 1970, 34, 595) est encore positif. On effectue donc un second couplage, dans les mêmes conditions, et on obtient cette fois un test de Kaiser négatif.

La peptidyl-résine est traitée par 1,5 ml d'un mélange TFA/phénol/EDT/thioanisole/eau (10 ml/0,75 g/0,25 ml/0,25 ml/0,5 ml) pendant 1 h 30. Le peptide est précipité dans un mélange éther éthylique/heptane refroidi à -20°C (2 x 20 ml). Le surnageant est éliminé. Le peptide précipité est resolubilisé dans 1 ml d'acide acétique. 5 ml d'eau sont ensuite ajoutés, la solution est dégazée à l'azote, congelée et lyophilisée. Le peptide brut obtenu est purifié par RP-HPLC sur une colonne C18 nucléosil. Eluant A: TFA 0,05% dans l'eau. Eluant B : eau/propan-2-ol (60/40) comprenant 0,05% de TFA. Le gradient est le suivant : de 0 à 60% de B en 60 minutes, le produit d'intérêt étant élué au bout de 23 minutes. Après purification, on obtient 19,9 mg de peptide pur (rendement : 29,7%), fonctionnalisé au niveau de son extrémité N-terminale par une fonction α-hydrazinoacétique. Son analyse par ES-MS est la suivante : [M+H]⁺ calculé : 1545,6 ; trouvé : 1545,4.

## Revendications

1. Procédé de couplage entre un peptide et au moins un composé A, de nature non peptidique, portant une fonction sélectionnée dans le groupe constitué par les fonctions acide carboxylique et les fonctions alcool, **caractérisé en ce que** ledit couplage comprend une étape de réalisation, en solution, d'un lien hydrazide entre ledit peptide et ledit composé A.

2. Procédé de couplage selon la revendication 1, **caractérisé en ce qu'**il comprend, pour réaliser ledit lien hydrazide, les étapes suivantes :
a) activation de la fonction portée par ledit composé A en une fonction réactive correspondante, respectivement sélectionnée dans le groupe constitué par les fonctions esters et les fonctions carbonate, lorsque le composé A porte respectivement une fonction acide carboxylique et une fonction alcool ; et
b) réaction, en solution et à un pH inférieur à 6, entre ledit composé A activé obtenu en a) et un peptide, totalement déprotégé, portant au moins un groupement hydrazine ou dérivé d'hydrazine soit au niveau de son extrémité N-terminale, soit au niveau de l'extrérnité de la chaîne latérale d'une lysine ou d'une ornithine éventuellement présente à un endroit quelconque de la séquence peptidique.

3. Procédé selon la revendication 2, **caractérisé en ce qu'**il comprend, en outre, une étape c) de purification du peptide modifié obtenu à l'étape b).

4. Procédé selon la revendication 2 ou la revendication 3, **caractérisé en ce que**, après l'étape a) d'activation de la fonction portée par le composé A, la fonction réactive correspondante portée par le composé A est sélectionnée dans le groupe constitué par les esters et les carbonates de succinimidyle, de sulfosuccinimidyle et d'aryle.

5. Procédé selon l'une quelconque des revendications 2 à 4, **caractérisé en ce que** ledit groupement dérivé d'hydrazine portée par le peptide est un groupement α-hydrazinoacétique.

6. Procédé selon la revendication 5, **caractérisé en ce que**, préalablement à l'étape b), ledit peptide est fonctionnalisé par un groupement α-hydrazinoacétique, soit au niveau de son extrémité N-terminale, soit au niveau de l'extrémité de la chaîne latérale d'une lysine ou d'une ornithine éventuellement présente à un endroit quelconque de la séquence peptidique, à l'aide de l'aride N,N'-tri(Boc)hydrazinoacétique ou de l'acide N,N'-di(Boc)hydrazinoacétique.

7. Procédé selon la revendication 6, **caractérisé en ce que** la fonctionnalisation dudit peptide par un groupement α-hydrazinoacétique est suivie d'une étape de purification dudit peptide fonctionnalisé par chromatographie liquide haute performance, à l'aide d'un éluant constitué d'un mélange eau/alcool, de préférence un mélange eau/isopropanol, comprenant de l'acide trifluoroacétique.

8. Procédé selon l'une quelconque des revendications précédentes, **caractérisé en ce que** ledit composé A est sélectionné dans le groupe constitué par les lipides, les sucres, les alcools et les marqueurs de fluorescence.

9. Procédé selon la revendication 8, **caractérisé en ce que** lesdits lipides sont sélectionnés dans le groupe constitué par les acides gras saturés, les acides gras insaturés et les stérols.

10. Procédé selon la revendication 9, **caractérisé en ce que** lesdits lipides sont sélectionnés dans le groupe constitué par l'acide palmitique, l'acide stéarique, l'acide cis-9,10-époxystéarique, l'acide oléique, l'acide linoléique et le cholestérol.

11. Peptide modifié, **caractérisé en ce qu'**il est essentiellement constitué d'un peptide lié, par un lien hydrazide, à au moins un composé A sélectionné dans le groupe constitué par les lipides, les sucres, les alcools et les marqueurs de fluorescence.

12. Peptide modifié selon la revendication 11, **caractérisé en ce qu'**il s'agit d'un oligopeptide essentiellement constitué d'un peptide lié, par un lien hydrazide, à au moins un lipide sélectionné dans le groupe constitué par les acides gras saturés, les acides gras insaturés et les stérols.

13. Peptide modifié selon la revendication 12, **caractérisé en ce qu'**il s'agit d'un oligopeptide essentiellement constitué d'un peptide lié, par un lien hydrazide, à au moins un lipide sélectionné dans le groupe constitué par l'acide palmitique, l'acide stéarique, l'acide cis-9,10-époxystéarique, l'acide oléique, l'acide linoléique et le cholestérol.

14. Vaccin synthétique, **caractérisé en ce qu'**il comprend au moins un peptide modifié selon l'une quelconque des revendications 11 à 13.

15. Réactif de diagnostic, **caractérisé en ce qu'**il comprend au moins un peptide modifié selon l'une quelconque des revendications 11 à 13.

16. Utilisation du résidu du procédé selon l'une quelconque des revendications 1 à 10 pour la préparation d'un médicament comprenant un principe actif de nature peptidique vectorisé, utile pour le ciblage cellulaire.

## Patentansprüche

1. Verfahren zur Kupplung zwischen einem Peptid und mindestens einer Verbindung A, mit nichtpeptidartiger Beschaffenheit, die eine Funktion, ausgewählt aus der Gruppe bestehend aus den Carbonsäurefunktionen und den Alkoholfunktionen, aufweist, **dadurch gekennzeichnet, dass** die Kupplung einen Schritt der Bildung einer Hydrazidbindung bzw. eines Hydrazidbindegliedes zwischen dem Peptid und der Verbindung A in Lösung umfasst.

2. Verfahren zur Kupplung nach Anspruch 1, **dadurch gekennzeichnet, dass es** zum Bilden der Hydrazidbindung bzw. des Hydrazidbindegliedes die folgenden Schritte umfasst:
a) Aktivierung der Funktion, welche die Verbindung A aufweist, zu einer entsprechenden reaktiven Funktion, ausgewählt aus der Gruppe bestehend aus den Esterfunktionen und den Carbonatfunktionen, wenn die Verbindung A eine Carbonsäurefunktion bzw. eine Alkoholfunktion aufweist; und
b) Reaktion in Lösung und bei einem pH unter 6 zwischen der bei a) erhaltenen aktivierten Verbindung A und einem Peptid, von dem alle Schutzgruppen entfernt sind und das mindestens eine Hydrazingruppe oder ein Hydrazinderivat entweder an seinem N-terminalen Ende oder am Ende der Seitenkette eines Lysins oder eines Ornithins aufweist, das gegebenenfalls an einem beliebigen Ort der Peptidsequenz vorhanden ist.

3. Verfahren nach Anspruch 2, **dadurch gekennzeichnet, dass** es außerdem einen Schritt c) der Reinigung des in Schritt b) erhaltenen modifizierten Peptids umfasst.

4. Verfahren nach Anspruch 2 oder Anspruch 3, **dadurch gekennzeichnet, dass** nach dem Schritt a) der Aktivierung der Funktion, welche die Verbindung A aufweist, die entsprechende reaktive Funktion, welche die Verbindung A aufweist, ausgewählt ist aus der Gruppe bestehend aus den Estern und den Carbonaten von Succinimidyl, von Sulfosuccinimidyl und von Aryl.

5. Verfahren nach einem der Ansprüche 2 bis 4, **dadurch gekennzeichnet, dass** die von Hydrazin abgeleitete Gruppe, welche das Peptid aufweist, eine α-hydrazinoessigsaure Gruppe ist.

6. Verfahren nach Anspruch 5, **dadurch gekennzeichnet, dass** vor dem Schritt b) das Peptid durch eine a-hydrazinoessigsaure Gruppe entweder an seinem N-terminalen Ende oder am Ende der Seitenkette eines Lysins oder eines Omithins, das gegebenenfalls an einem beliebigen Ort der Peptidsequenz vorhanden ist, mit Hilfe von N,N'-Tri(Boc)hydrazinoessigsäure oder von N,N'-Di(Boc)hydrazinoessigsäure funktionalisiert wird.

7. Verfahren nach Anspruch 6, **dadurch gekennzeichnet, dass** auf die Funktionalisierung des Peptids durch eine α-hydrazinoessigsaure Gruppe ein Schritt der Reinigung des funktionalisierten Peptids durch Hochleistungsflüssigchromatografie mittels eines Elutionsmittels, das aus einem Wasser/Alkohol-Gemisch, vorzugsweise einem Wasser/lsopropanol-Gemisch, das Trifluoressigsäure umfasst, besteht, folgt.

8. Verfahren nach einem der vorhergehenden Ansprüche, **dadurch gekennzeichnet, dass** die Verbindung A ausgewählt ist aus der Gruppe bestehend aus den Lipiden, den Zuckern, den Alkoholen und den Fluoreszenzmarkem.

9. Verfahren nach Anspruch 8, **dadurch gekennzeichnet, dass** die Lipide ausgewählt sind aus der Gruppe bestehend aus den gesättigten Fettsäuren, den ungesättigten Fettsäuren und den Sterolen.

10. Verfahren nach Anspruch 9, **dadurch gekennzeichnet, dass** die Lipide ausgewählt sind aus der Gruppe bestehend aus Palmitinsäure, Stearinsäure, cis-9,10-Epoxystearinsäure, Ölsäure, Linolsäure und Cholesterin.

11. Modifiziertes Peptid, **dadurch gekennzeichnet, dass** es im Wesentlichen besteht aus einem Peptid, das durch eine Hydrazidbindung bzw. ein Hydrazidbindeglied an wenigstens eine Verbindung A gebunden ist, die ausgewählt ist aus der Gruppe bestehend aus den Lipiden, den Zuckern, den Alkoholen und den Fluoreszenzmarkem.

12. Modifiziertes Peptid nach Anspruch 11, **dadurch gekennzeichnet, dass** es sich um ein Oligopeptid handelt, das im Wesentlichen besteht aus einem Peptid, das durch eine Hydrazidbindung bzw. ein Hydrazidbindeglied an mindestens ein Lipid gebunden ist, das ausgewählt ist aus der Gruppe bestehend aus den gesättigten Fettsäuren, den ungesättigten Fettsäuren und den Sterolen.

13. Modifiziertes Peptid nach Anspruch 12, **dadurch gekennzeichnet, dass** es sich um ein Oligopeptid handelt, das im Wesentlichen besteht aus einem Peptid, das durch eine Hydrazidbindung bzw. ein Hydrazidbindeglied an mindestens ein Lipid gebunden ist, das ausgewählt ist aus der Gruppe bestehend aus Palmitinsäure, Stearinsäure, cis-9,10-Epoxystearinsäure, Ölsäure, Linolsäure und Cholesterin.

14. Synthetischer Impfstoff, **dadurch gekennzeichnet, dass** er mindestens ein modifiziertes Peptid nach einem der Ansprüche 11 bis 13 umfasst.

15. Diagnosereagenz, **dadurch gekennzeichnet, dass es** mindestens ein modifiziertes Peptid nach einem der Ansprüche 11 bis 13 umfasst.

16. Verwendung des Produkts des Verfahrens nach einem der Ansprüche 1 bis 10 zum Herstellen eines Arzneimittels, das einen Wirkstoff mit vektorisierter peptidartiger Beschaffenheit umfasst, das für eine gezielte Ansteuerung von Zellen brauchbar ist.

## Claims

1. Method of coupling between a peptide and at least one compound A of non-peptidic nature carrying a function selected from the group consisting of carboxylic acid functions and alcohol functions, **characterised in that** the said coupling comprises a step of producing, in solution, a hydrazide link between the said peptide and the said compound A.

2. Method of coupling according to claim 1, **characterised in that** it comprises, for producing the said hydrazide link, the following steps:
a) activation of the function carried by the said compound A to form a corresponding reactive function respectively selected from the group consisting of ester functions and carbonate functions when compound A carries, respectively, a carboxylic acid function and an alcohol function; and
b) reaction, in solution and at a pH of less than 6, between the said activated compound A obtained in a) and an entirely unprotected peptide carrying at least one hydrazine group or group derived from hydrazine either at its N-terminal end or at the end of the side chain of a lysine or ornithine optionally present at any location in the peptide sequence.

3. Method according to claim 2, **characterised in that** it comprises, in addition, a step c) of purifying the modified peptide obtained in step b).

4. Method according to claim 2 or claim 3, **characterised in that**, after step a) of activating the function carried by compound A, the corresponding reactive function carried by compound A is selected from the group consisting of esters and carbonates of succinimidyl, sulfosuccinimidyl and aryl.

5. Method according to any one of claims 2 to 4, **characterised in that** the said group derived from hydrazine carried by the peptide is an α-hydrazinoacetic group.

6. Method according to claim 5, **characterised in that**, prior to step b), the said peptide is functionalised by an α-hydrazinoacetic group, either at its N-terminal end or at the end of the side chain of a lysine or ornithine optionally present at any location in the peptide sequence, using N,N'-tri(Boc)hydrazinoacetic acid or N,N'-di(Boc)hydrazinoacetic acid.

7. Method according to claim 6, **characterised in that** the functionalisation of the said peptide by an α-hydrazinoacetic group is followed by a step of purifying the said functionalised peptide by high-performance liquid chromatography, using an eluant composed of a water/alcohol mixture, preferably a water/isopropanol mixture, including trifluoroacetic acid.

8. Method according to any one of the preceding claims, **characterised in that** the said compound A is selected from the group consisting of lipids, sugars, alcohols and fluorescence markers.

9. Method according to claim 8, **characterised in that** the said lipids are selected from the group consisting of saturated fatty acids, unsaturated fatty acids and sterols.

10. Method according to claim 9, **characterised in that** the said lipids are selected from the group consisting of palmitic acid, stearic acid, cis-9,10-epoxystearic acid, oleic acid, linoleic acid and cholesterol.

11. Modified peptide, **characterised in that** it is composed substantially of a peptide linked by way of a hydrazide link to at least one compound A selected from the group consisting of lipids, sugars, alcohols and fluorescence markers.

12. Modified peptide according to claim 11, **characterised in that** it is an oligopeptide composed substantially of a peptide linked by way of a hydrazide link to at least one lipid selected from the group consisting of saturated fatty acids, unsaturated fatty acids and sterols.

13. Modified peptide according to claim 12, **characterised in that** it is an oligopeptide composed substantially of a peptide linked by way of a hydrazide link to at least one lipid selected from the group consisting of palmitic acid, stearic acid, cis-9,10-epoxystearic acid, oleic acid, linoleic acid and cholesterol.

14. Synthetic vaccine, **characterised in that** it comprises at least one modified peptide according to any one of claims 11 to 13.

15. Diagnostic reagent, **characterised in that** it comprises at least one modified peptide according to any one of claims 11 to 13.

16. Use of the result of the method according to any one of claims 1 to 10 in the preparation of a medicament comprising a vectorised active ingredient of peptidic nature for use in cell targeting.
